(19) Europäisches Patentamt
European Patent Office
Office européen des brevets

(11) **EP 3 088 918 A2**

(12) **EUROPEAN PATENT APPLICATION**

(43) Date of publication:
**02.11.2016 Bulletin 2016/44**

(51) Int Cl.:
***G01T 1/17*** *(2006.01)*

(21) Application number: **16166380.2**

(22) Date of filing: **25.03.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.03.2012 US 201261616055 P**

(62) Document number(s) of the earlier application(s) in accordance with Art. 76 EPC:
**13723960.4 / 2 831 630**

(71) Applicant: **Koninklijke Philips N.V.**
**5656 AE Eindhoven (NL)**

(72) Inventors:
• **DAERR, Heiner**
**5656 AE Eindhoven (NL)**
• **ROESSL, Ewald**
**5656 AE Eindhoven (NL)**

(74) Representative: **Steffen, Thomas**
**Philips Intellectual Property & Standards**
**High Tech Campus 5**
**5656 AE Eindhoven (NL)**

Remarks:
This application was filed on 21-04-2016 as a divisional application to the application mentioned under INID code 62.

(54) **CONVENTIONAL IMAGING WITH AN IMAGING SYSTEM HAVING PHOTON COUNTING DETECTORS**

(57) An imaging system (600) includes a radiation source (608) that emits polychromatic radiation that traverses an examination region and a detector array (610) located opposite the radiation source, across the examination region, which includes a paralyzable photon counting detector pixel (611) that detects photons of the radiation that traverse the examination region and illuminate the detector pixel and that generates a signal indicative of each detected photon. An output photon count rate to input photon count rate map (626) includes at least one map which maps multiple input photon count rates of the detector pixel to a single output photon count rate of the detector pixel, and an input photon count rate determiner (624) identifies one input photon count rate of the multiple input photon count rates of the map as a correct input photon count rate for the detector pixel. A reconstructor that reconstructs the signal based on the identified input photon count rate.

FIG. 6

EP 3 088 918 A2

## Description

The following generally relates to conventional imaging with an imaging system having photon counting detectors and finds particular application to computed tomography (CT); however, the following is also amenable to other imaging including, but not limited to, x-ray and mammography.

A conventional (integrating) computed tomography (CT) scanner includes an x-ray tube supported by a rotating frame. The rotating frame and hence the x-ray tube rotate around an examination region, and the x-ray tube emits polychromatic radiation that traverses the examination region and a subject and/or object disposed therein. A radiation sensitive detector is located opposite the x-ray tube, across the examination region, and detects radiation that traverses the examination region and the subject and/or object. The radiation sensitive detector includes a one or two dimensional array of integrating detector pixels, such as scintillator/photosensor based pixels along with corresponding integrating electrical circuitry. Generally, the scintillator produces light in response to absorbing incident photons, the photosensor produces electrical charge indicative of the absorbed photons in response to receiving the light, and the integrating electrical circuitry accumulates the charge and generates projection data indicative of the detected radiation.

A reconstructor reconstructs the projection data and generates volumetric image data indicative of the subject and/or object. An image processor can be used to process the volumetric image data and generate one or more images indicative of the subject and/or object. Generally, the volumetric image data / image include voxels / pixels that are represented in terms of gray scale values corresponding to relative radiodensity. Such information reflects the x-ray attenuation characteristics of the scanned subject and/or object, and generally shows structure such as anatomical structures within a subject, physical structures within an inanimate object, etc. However, since the absorption of a photon by a material is dependent on the energy of the photon traversing the material, the detected radiation also includes spectral information, which provides additional information indicative of an elemental composition (e.g., atomic number) of the tissue and/or material. Unfortunately, the volumetric image data generated in conventional (integrating) CT does not reflect the spectral characteristics, as the signal generated by the detector is proportional to the energy fluence integrated over the energy spectrum.

A spectral CT scanner, in addition to the components discussed above, includes one or more components that capture the spectral characteristics of the detected radiation. An example of such a component(s) is a photon-counting detector including a direct conversion semiconductor material such as Cadmium Telluride (CdTe), Cadmium Zinc Telluride (CZT) or the like, and corresponding processing circuitry. With such a detector, each pixel produces an electrical signal for each photon it detects, and the electrical signal is indicative of the energy of that photon. An amplifier amplifies the signal, and a signal shaper shapes the amplified signal, forming an electrical pulse having a height or peak indicative of the energy of the photon. A discriminator compares the amplitude of the pulse with one or more energy thresholds that are set in accordance with different energy levels corresponding to mean emission levels of the x-ray tube. A counter counts, for each threshold, the number of times the amplitude exceeds the threshold, and a binner bins or assigns a detected photon to an energy range based on the counts. The resulting energy-resolved detected radiation can be reconstructed using a spectral and/or conventional reconstruction algorithm, producing spectral and/or conventional image data and/or images.

The behavior of a CdTe or CZT based photon-counting detector can be modeled with sufficient accuracy either using a paralyzable detector model or a non-paralyzable detector model, depending on the sensor and detector electronics. Below we discuss difficulties in the reconstruction of the incident rate from ambiguous measurements of the output count rate in a detector system that can be described by the paralyzable detector model. Generally, a paralyzable detector is one in which each detected photon has a non-zero (e.g., 10 to 100 nanosecond) resolving or dead time such that if another photon is detected during the dead time, the detector will not be able to resolve the individual photon detections and the resulting pulse will have amplitude depending on the amplitudes of the individual pulses and the time difference between their arrivals. The dead time is a function of a width of the pulses generated by the shaper, and the output photon count rate of a paralyzable detector is a function of a number of incident x-ray photons per time (the input photon count rate) and the dead time. The output photon count rate can be expressed as shown in EQUATION 1:

$$\text{EQUATION 1:}$$
$$m = r \cdot e^{-r \cdot \tau},$$

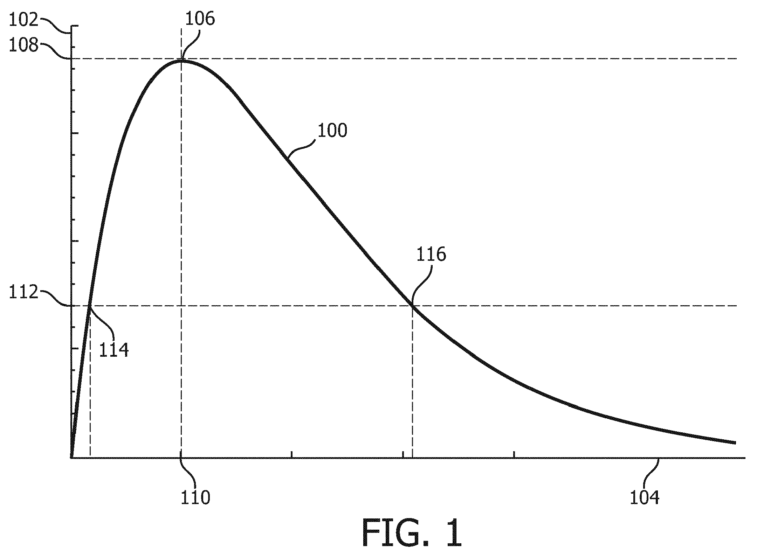

where m represents the output photon count rate, $r$ represents the input photon count rate, and $\tau$ represents the dead time. An example of this behavior is shown graphically in FIGURE 1 through curve 100, where a y-axis 102 represents the average output photon count rate m and an x-axis 104 represents the input photon count rate $r$ as a function of the dead time $\tau$. In FIGURE 1, a peak 106 of the curve 100 represents a maximum output photon count rate 108, which occurs at an input photon count rate 110 that corresponds to $r_{MAX} = 1/\tau$. For output photon count rates (e.g., an output photon count rate 112) less than the maximum output photon count rate 108, there exist two possible input photon count rates (e.g., input photon count rate 114 and input photon count rate 116), one less than $r_{MAX}$ and one greater than $r_{MAX}$.

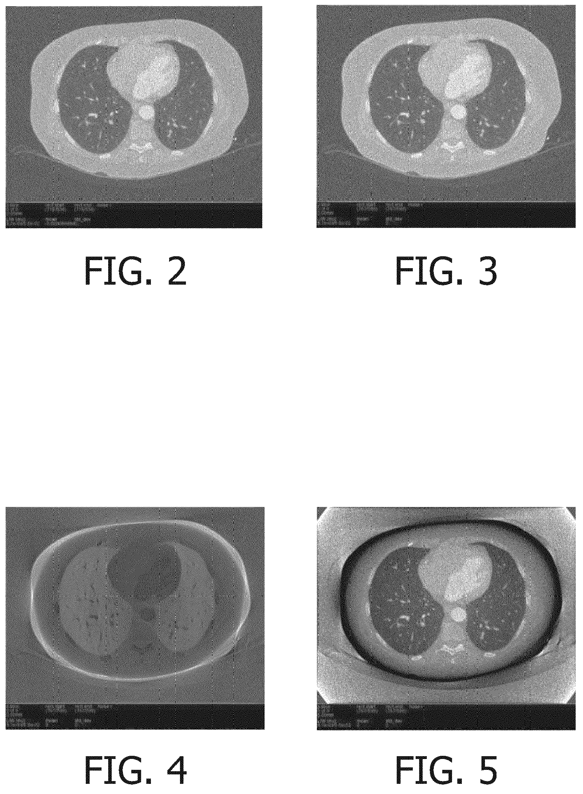

In order to correctly reconstruct the data corresponding to the output photon count rate 112 to generate conventional image data, the correct input photon count rate 114 or 116 corresponding to the data needs to be known. Reconstructing the data with the incorrect input photon count rate introduces artifact into the images, which may render the images unsuitable for diagnostic purposes. By way of example, FIGURE 2 shows an image reconstructed from simulated data for a conventional integrating detector, and FIGURE 3 shows an image reconstructed from simulated data for a counting detector where the correct input photon count rate is known. Note that visually the image of FIGURE 3 is very similar to the image of FIGURE 2, but has a slightly higher noise level. In contrast, FIGURE 4 shows an image reconstructed from simulated data for a counting detector under the assumption that $r$ is greater than $r_{MAX}$ in all cases, and FIGURE 5 shows an image reconstructed from simulated data for a counting detector under the assumption that $r$ is smaller than $r_{MAX}$ in all cases. FIGURES 4 and 5 visually show that reconstructing images using the incorrect input photon count rate introduces artifact.

Aspects described herein address the above-referenced problems and others.

In one aspect, an imaging system includes a radiation source that emits polychromatic radiation that traverses an examination region and a detector array located opposite the radiation source, across the examination region, which includes a paralyzable photon counting detector pixel that detects photons of the radiation that traverse the examination region and illuminate the detector pixel and that generates a signal indicative of each detected photon. An output photon count rate to input photon count rate map includes at least one map which maps multiple input photon count rates of the detector pixel to a single output photon count rate of the detector pixel, and an input photon count rate determiner identifies one input photon count rate of the multiple input photon count rates of the map as a correct input photon count rate for the detector pixel. A reconstructor that reconstructs the signal based on the identified input photon count rate.

In another aspect, a method includes receiving an output signal of a paralyzable photon counting detector pixel that is receiving photons at an input photon count rate. The method further includes determining an output photon count rate of the detector pixel. The method further includes identifying an input photon count rate, from multiple candidate input photon count rates for the output photon count rate, as the input photon count rate corresponding to the detector pixel and the output photon count rate. The method further includes reconstructing the output signal based on the identified input photon count rate.

The invention may take form in various components and arrangements of components, and in various steps and arrangements of steps. The drawings are only for purposes of illustrating the preferred embodiments and are not to be construed as limiting the invention.

FIGURE 1 graphically illustrates example of the output photon count rate behavior of a paralyzable photon counting detector as a function of input photon count rate and pulse shaping dead time.

FIGURE 2 illustrates an image produced based on simulated data from a conventional detector.

FIGURE 3 illustrates a conventional image produced based on simulated data from a paralyzable photon counting detector and using the correct input photon count rate.

FIGURE 4 illustrates a conventional image produced based on simulated data from a paralyzable photon counting detector and using an input photon count rate that is less than $r_{MAX}$ when the correct input photon count rate is greater than $r_{MAX}$.

FIGURE 5 illustrates a conventional image produced based on simulated data from a paralyzable photon counting detector and using an input photon count rate that is greater than $r_{MAX}$ when the correct input photon count rate is less than $r_{MAx}$.

FIGURE 6 schematically illustrates an example CT imaging system having a photon counting detector and in connection with an input photon count rate determiner.

FIGURE 7 schematically illustrates an example of the input photon count rate determiner of FIGURE 6 which uses a time over threshold value to determine the correct input photon count rate.

FIGURE 8 graphically illustrates an example of a measured output photon count rate of a detector pixel for an integration period for a lower input photon count rate.

FIGURE 9 graphically illustrates an example of a measured output photon count rate, which has the same value as that of FIGURE 8, of a detector pixel for an integration period for a relatively higher input photon count rate.

FIGURE 10 graphically illustrates the amount of time pulses are above a given threshold as a function of input photon count rate.

FIGURE 11 schematically illustrates an example of the input photon count rate determiner of FIGURE 6 which uses a pulse pile-up count to determine the correct input photon count rate.

FIGURE 12 schematically illustrates an example of the input photon count rate determiner of FIGURE 6 which uses information from at least two different size detector pixels to determine the correct input photon count rate.

FIGURE 13 schematically illustrates an example of the input photon count rate determiner of FIGURE 6 which uses at least two different shaping times to generate information to determine the correct input photon count rate.

FIGURE 14 schematically illustrates an example of the input photon count rate determiner of FIGURE 6 which uses information generated from using at least two different flux rates to generate information to determine the correct input photon count rate.

FIGURE 15 graphically illustrates an example of the counts in a bin as a function of the input photon count rate in connection with FIGURE 1.

FIGURE 16 illustrates an example method.

The following describes an approach for generating conventional images with a spectral imaging system having paralyzable photon-counting detectors where the images have an image quality that is similar to an image quality of images generated with a conventional (non-spectral) imaging system.

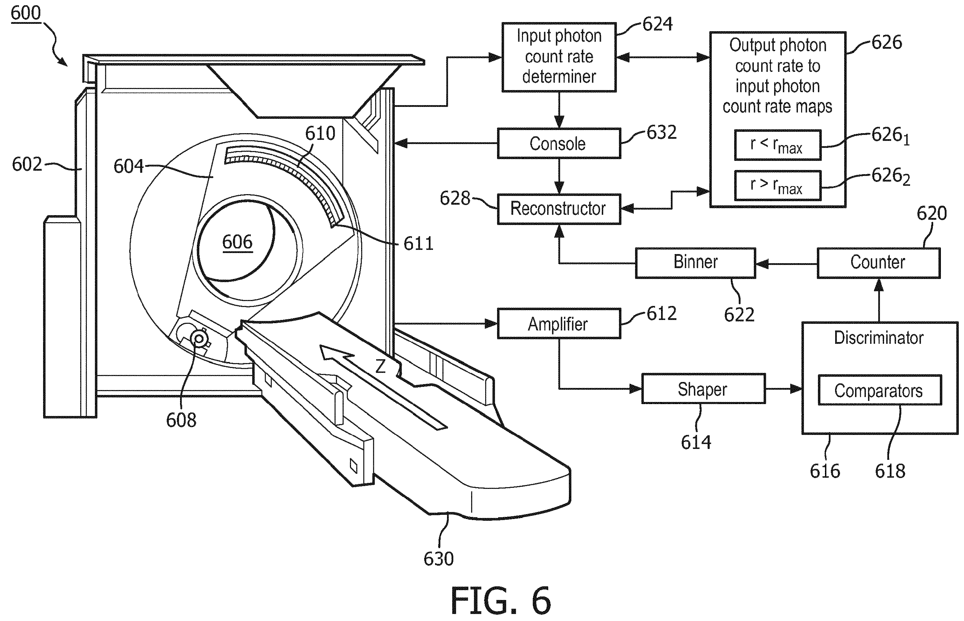

Initially referring to FIGURE 6, an example spectral CT scanner 600 is illustrated. The CT scanner 600 includes a generally stationary gantry 602 and a rotating gantry 604, which is rotatably supported by the stationary gantry 602 and rotates around an examination region 606 about a z-axis. A radiation source 608, such as an x-ray tube, is rotatably supported by the rotating gantry 604, rotates with the rotating gantry 604, and emits polychromatic radiation that traverses the examination region 606.

A radiation sensitive detector array 610 subtends an angular arc opposite the radiation source 608 across the examination region 606. The radiation sensitive detector array 610 detects radiation traversing the examination region 606 and generates a signal indicative thereof for each detected photon. In the illustrate embodiment, the radiation sensitive detector array 610 is a photon-counting detector array with a one or two dimensional array of photon-counting detector pixels 611 that include direction conversion material such as CdTe, CZT, and/or other paralyzable direct conversion material.

For each detector pixel 611, an optional amplifier 612 amplifies the signal. A shaper 614 processes the amplified signal and generates a pulse such as voltage or other pulse indicative of the energy of the detected photon. A discriminator 616 energy discriminates the pulse. In the illustrated example, the energy discriminator 616 includes one or more comparators 618 that compare the amplitude of the pulse with different energy thresholds, which correspond to different energies of interest. The discriminator 616 produces an output (e.g., high or low, 0 or 1, etc.) that indicates whether, for each threshold, the amplitude exceeds the threshold.

A counter 620 increments a count value for each threshold based on the output of the discriminator 616. For instance, when the output of the comparator 618 for a particular threshold indicates that the amplitude of the pulse exceeds the corresponding threshold, the count value for that threshold is incremented. A binner 622 energy bins the signals and, hence, the photons into two or more energy bins based on the counts. Generally, an energy bin encompasses an energy range or window. For example, a bin may be defined for the energy range between two thresholds, where a photon resulting in a count for the lower threshold but not for higher threshold would be assigned to that bin.

An input photon count rate determiner 624 determines the correct input photon count rate, from multiple candidate input photon count rates, for each detector pixel 611 each integration period from at least an output photon count rate to input photon count rate map 626, which includes a first sub-map $626_1$ corresponding to $r < r_{MAX}$ (FIGURE 1) and a second sub-map $626_2$ corresponding to $r > r_{MAX}$ (FIGURE 1). The map 626 can be generated based on an air scan using different and known flux rates and/or a series of calibration scans using various thicknesses of tissue equivalent materials of known attenuation properties.

As described in greater detail below, the input photon count rate determiner 624 determines the correct input photon count rate based on one or more approaches including, but not limited to, an amount time pulses generated in response to detecting photons exceed a threshold during an integration period, a number of detected pulse pile-ups in an integration period, a ratio of input photon count rates for different detectors having different and known radiation sensitive areas, a ratio of input photon count rates for different radiation source emission fluxes, estimates based on a sinogram, a distribution of count values of an energy bin, and/or otherwise.

A reconstructor 628 reconstructs the data based on the input photon count rate determined by the input photon count rate determiner 624, generating volumetric image data, which can be processed to produce one or more conventional images. As discussed herein, reconstructing the data using the correct one of the multiple candidate input photon count rates mitigates artifact introduced by reconstructing the data based on an incorrect one of the input photon count rates, and facilitates generating images having an image quality comparable to an image quality of images generated with a conventional CT scanner.

A subject support 630, such as a couch, supports an object or subject in the examination region 606. A general-purpose computing system or computer serves as an operator console 632. The console 632 includes a human readable output device such as a monitor and an input device such as a keyboard, mouse, etc. Software resident on the console 632 allows the operator to interact with and/or operate the scanner 600 via a graphical user interface (GUI) or otherwise.

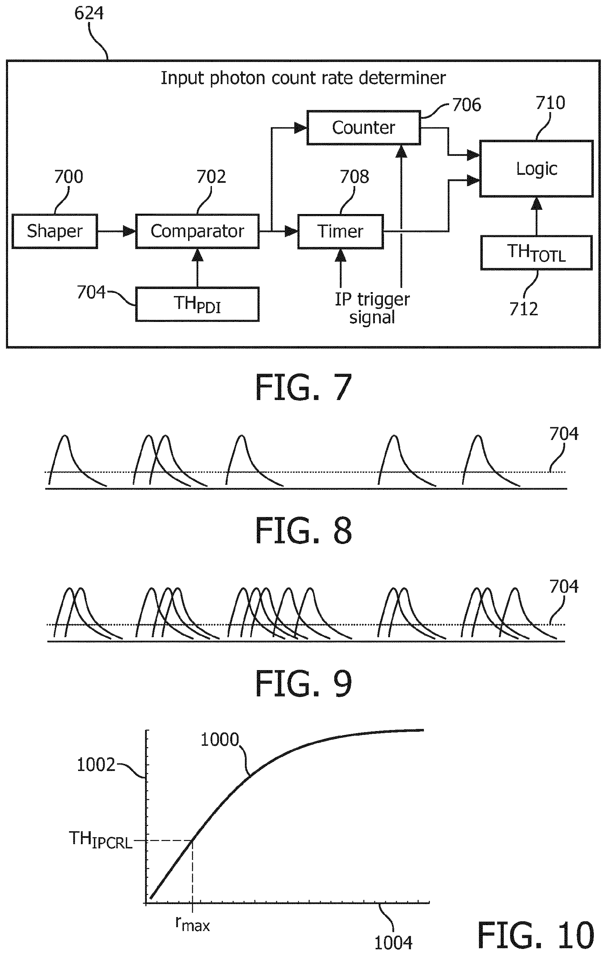

FIGURE 7 illustrates a non-limiting example of the input photon count rate determiner 624 for one of the detector pixels 611.

A shaper 700 receives the output signal from the detector pixel 611 and generates, for each detected photon, a pulse such as voltage or other pulse having a peak height indicative of an energy of the detected photon. A comparator 702 compares an amplitude of the pulses with a photon detection identifying threshold (TH$_{PDI}$) 704 and produces an output (e.g., high or low, 0 or 1, etc.) that indicates whether the amplitude exceeds the threshold. In one instance, the value of the threshold 704 is at or just above a noise level of the detector pixel 611, which facilitates discriminating between detected photons and noise.

A counter 706 increments a count value each time the output of the comparator 702 transitions from indicating the output is below the threshold 704 to indicating the output has exceeded the threshold 704. As discussed herein, such a transition may be indicative of an individual photon detection or multiple piled up (overlapping) photons. The counter 706 resets each integration period, for example, upon receiving an integration period (IP) trigger signal, and outputs the count value, which is a measure of the output photon count rate for the corresponding integration period. The integration period time can be measured, or a predetermined static value can be used.

Briefly turning to FIGURES 8 and 9, examples of two different input photon count rates resulting in a same measured output photon count rate, due to pulse pile-up, are shown. In FIGURE 8, a lower input photon count rate of six photons within a predetermined period of time (e.g., an integration period) results in an output photon count rate of five photons with pulses for two of the detected photons overlapping such that they cannot be individually resolved. In FIGURE 9, a higher input photon count rate of fifteen photons within the same period of time also results in an output photon count rate of five photons within the same period of time due to overlapping pulses which cannot be individually resolved. As a result, the correct input photon count rate of the multiple candidate input photon count rates of the map 626 cannot be determined from the measured output photon count rate alone.

Returning to FIGURE 7, a timer 708 times the amount of time the amplitude of the output of the comparator 702 indicates the threshold 704 is exceeded. That is, the timer 708 is activated in response to the output of the comparator 702 rising to or above the threshold 704 and continues until the output falls below the threshold 704. The timer 708 resets each subsequent integration period, for example, upon receiving the IP trigger signal, and outputs a time over threshold value. Briefly turning to FIGURE 10, a time over threshold curve 1000 is graphically illustrated as a function of input photon count rate, in which a y-axis 1002 represents time over threshold within one integration period, and an x-axis 1004 represents the input photon count rate. As shown, the time over threshold increases monotonically, unlike the measured output photon count rate (FIGURE 1), even once $r_{max}$ is reached and exceeded.

Returning to FIGURE 7, logic 710 receives the time over threshold value, compares it with an input photon time-over-threshold level (TH$_{TOTL}$) 712, and identifies the sub-map 626$_1$ as the correct sub-map in response to the time over threshold value falling under the threshold 712 or the sub-map 626$_2$ as the correct sub-map in response to the time over threshold value meeting or exceeding the threshold 712. The logic 710 populates a two-dimensional matrix, which corresponds to the sinogram, which indicates the correct sub-map for each data point in the sinogram.

In this embodiment, the logic 710 utilizes the matrix, the measured output photon count rate, and the map 626 to obtain the input photon count rate for reconstruction. In another embodiment, the reconstructor 628, the console 632 and/or other component utilizes the matrix, the measured output photon count rate, and the map 626 to obtain the input photon count rate for reconstruction. In such an embodiment, the counter 706 can be omitted from the input photon count rate determiner 624. For sake of brevity and clarity, the counter 706 is not shown in the following embodiments, but can be included therewith. Wherein included, the logic in the following embodiments can employ the output thereof as discussed in connection with FIGURE 7 to determine the input photon count rate and/or otherwise.

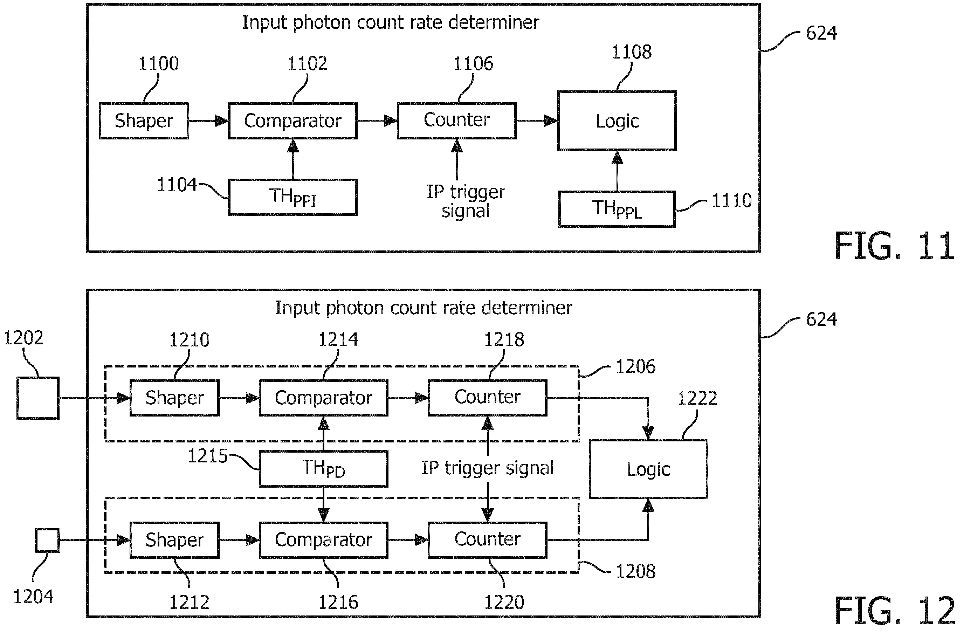

FIGURE 11 illustrates another non-limiting example of the input photon count rate determiner 624. In this example, a shaper 1100 and a comparator 1102 operate substantially similar to the shaper 700 and the comparator 702 of FIGURE 7. However, in this example, the comparator 1102 compares the amplitude of the output of the shaper 1100 with pulse pile-up identifier threshold (TH$_{PPI}$) 1104, which has a value that is larger than the radiation source emission voltage. As such, the amplitude of the output of the shaper 1100 will only exceed TH$_{PPI}$ when there is a pulse pile-up event in which individual pulses overlap and combine to produce an amplitude that exceeds TH$_{PPI}$. A counter 1106 counts pulse pile-up events and the logic 1108 compares the count value with a pulse pile-up level threshold (TH$_{PPL}$) 1110. Logic 1108 operates substantially similar to the logic 710 of FIGURE 7 and at least identifies the correct sub-map based on the comparison.

FIGURE 12 schematically illustrates a non-limiting example of the input photon count rate determiner 624 in connection with at least two detector pixels 1202 and 1204, which have different size radiation sensitive areas. In this example, a first processing chain 1206 processes data corresponding to the detector pixel 1202 and a second processing chain 1208 processes data corresponding to a detector pixel 1204. The processing chains 1206 and 1208 respectively include shapers 1210 and 1212, comparators 1214 and 1216, and counters 1218 and 1220, which operate substantially similar to the shaper 700, the comparator 702, and the counter 706 of FIGURE 7. The comparators 1214 and 1216 can employ a threshold (TH) similar to that of FIGURE 7 or 11, or a different threshold.

In this example, the detector pixel 1202 has a radiation sensitive area that is x (where x is a real number greater than zero) times the size of the radiation sensitive area of the detector pixel 1204. Both detector pixels 1202 and 1204 are irradiated by the same input photon count rate (IPCR) per mm$^2$, but due to their different size radiation sensitive areas, they will see different IPCR'ss; namely, the smaller area detector pixel 1204 will see IPCR$_s$ and the larger area detector pixel 1202 will see IPCR$_b$ = x·IPCR$_s$. As such, there will be two different output photon count rates (OPCR) measured by the counters 1218 and 1220, namely $m_b$ and $m_{s,}$. IPCR$_s$ can be expressed as shown in EQUATION 2:

EQUATION 2:

$$\text{IPCR}_s = \frac{\ln\left(\frac{ms}{xmb}\right)}{(1-x)\tau},$$

and IPCR$_b$ can be expressed as shown in EQUATION 3:

EQUATION 3:

$$\text{IPCR}_b = x \cdot \text{IPCR}_s.$$

In this example, the output photon count rate to input photon count rate maps 626 can include separate maps for the different size detector pixels 1202 and 1204, or separate maps 626 can be created for the different size detector pixels 1202 and 1204. Logic 1222 determine the correct input photon count rate from the output photon count rate to input photon count rate maps 626 of the detector pixels 1202 and 1204 and the measurements $m_b$ and $m_s$ by selecting the input photon count rates that satisfies EQUATION 3. Where the smaller area detector pixel 1204 always detects an incoming rate below $r_{max}$, a well-defined solution exists for the input photon count rate.

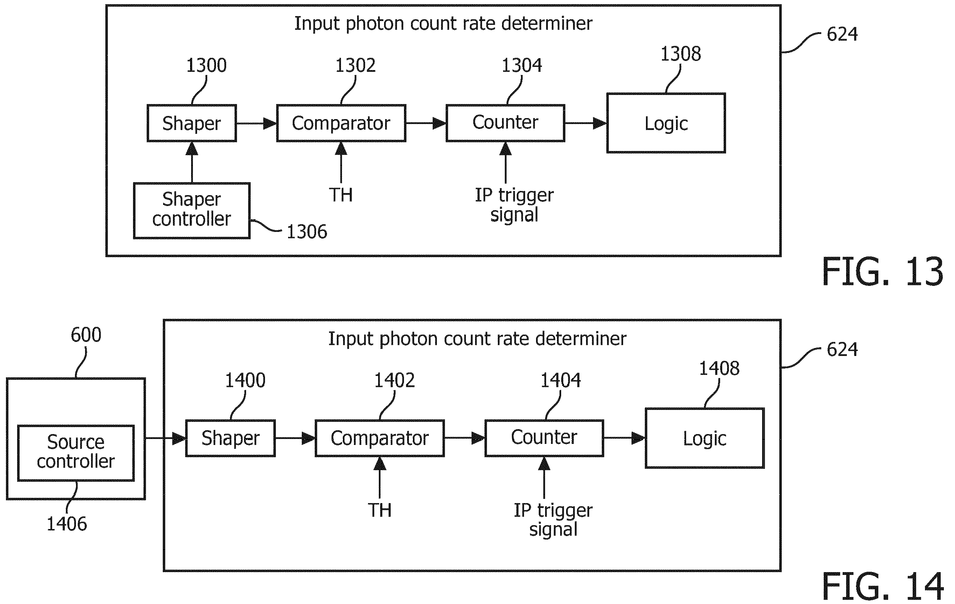

FIGURE 13 schematically illustrates another non-limiting example of the input photon count rate determiner 624. In this example, a shaper 1300, a comparator 1302, and a counter 1304 operate substantially similar to the shaper 700, the comparator 702, and the counter 706 of FIGURE 7. However, in this example, the input photon count rate determiner 624 further includes a shaper controller 1306, which switches a shaping time of the shaper 1300 between at least two different shaping times each integration period. By way of non-limiting example, in one instance, the shaper controller 1306 switches the shaping time between $\tau_1$ and $\tau_2$. As a result, there will be two curves 100 (FIGURE 1) and two different $r_{max's}$ for each detector pixel, one for $\tau_1$ and $\tau_2$. In addition, there will be two different output photon count rates (e.g., $m_1$ and $m_2$). The logic 1308 determines the input photon count rate based on $\tau_1, \tau_2, m_1$ and $m_2$ as shown in EQUATION 4:

EQUATION 4:

$$\text{IPCR} = \frac{\ln\left(\frac{m_2}{m_1}\right)}{\tau_1 - \tau_2}$$

where $m_i$ = IPCR exp(-IPCR $\tau_i$) and $i$ = 1,2. In one instance, the value of one of $\tau_1$ or $\tau_2$ is such that $r_{max}$ is greater than the input photon count rate, which allows for a well-defined solution for the input photon count rate. In an alternative embodiment, a second counter can be implemented without energy discrimination and a shorter $\tau$.

In a variation of the above, the shaper 1300 includes a plurality of sub-shapers, and at least two of the sub-shapers have different static or switchable shaping times. In one instance, at least two of the plurality of sub-shapers share the comparator 1302 and/or the counter 1304. In another instance, the comparator 1302 and/or the counter 1304 respectively include two or more sub-comparators and/or sub-counters, and the output of the at least two of the plurality of sub-shapers is processed by different sub-comparators and/or counters. In yet another variation, the input photon count rate determiner 624 includes two or more data pipelines or chains, each including a different shaper 1300, a different comparator 1302 and/or a different counter 1304.

FIGURE 14 schematically illustrates another non-limiting example of the input photon count rate determiner 624. In this example, a shaper 1400, a comparator 1402, and a counter 1404 operate substantially similar to the shaper 700, the comparator 702, and the counter 706 of FIGURE 7. In this example, the imaging system 600 further includes a source controller 1406, which is configured to switch the x-ray flux of the radiation source 608 between at least two different levels during scanning. Generally, a reduction of the incoming rate leads to an a decrease of the output count rate $m$, if the rate $r$ is far below $r_{max}$, and an increase of the output count rate $m$, if $r$ is far above $r_{max}$, compared to the signal in the long time period at a high flux. Similar to using two pixels with different areas described above in connection with FIGURE 12, each of the at least two different fluxes will have a corresponding different output photon count rate (e.g., $m_h$ and $m_l$), and logic 1408 can determine the input photon count rates based on $m_1$ and $m_s$ as shown in EQUATIONS 5 and 6:

EQUATION 5:

$$\text{IPCR}_l = \frac{\ln\left(\frac{m_s}{x\,m_l}\right)}{(1-x)\tau},$$

and

## EQUATION 6:

$$IPCR_s = x \cdot IPCR_l,$$

where x represents the ratio of the two different flux rates.

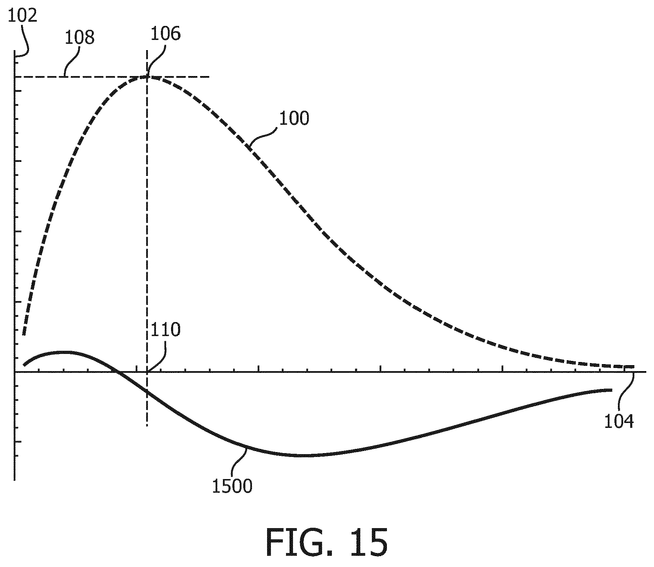

In another example, a distribution of the counted numbers of photons in an energy bin is used to estimate the amount of pile-ups, which can be used as a measure for the incoming rate $r$. This is described in connection with FIGURE 15, which includes FIGURE 1 and additionally a second curve 1500, which represents the counts in bin, which correspond to a difference between the two counters defining the bin. Note that the curve 1500 increases with increasing IPCR up to a point at which the curve 1500 begins to decreases due to increasing pulse pile up. As such, the count value of one or more energy bins can be monitored and used to facilitate determining the correct input photon count rate for each detector pixel within each integration period.

In another example, the decision for every detector pixel is made by looking at the sinogram and using prior knowledge. For example, in one instance, high flux conditions can be assumed to be at a periphery of the sinogram and low flux conditions can be assumed to be at a center region of the sinogram.

In another embodiment, a combination of the approaches discussed herein and/or one or more other approaches can be used to facilitate determining the input photon count rate for each detector pixel within each integration period.

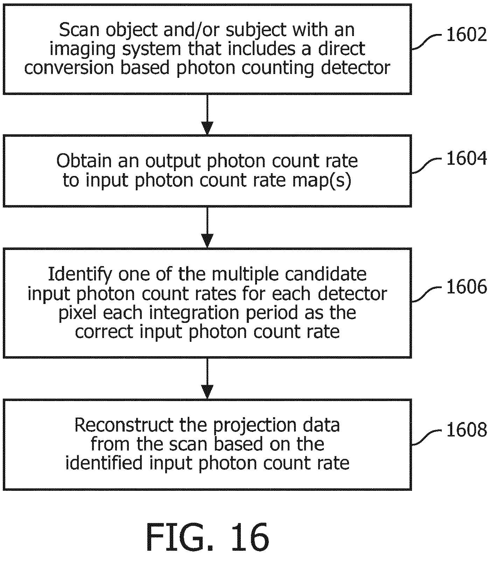

FIGURE 16 illustrates an example method in accordance with the embodiments described herein.

It is to be appreciated that the ordering of the acts in the methods described herein is not limiting. As such, other orderings are contemplated herein. In addition, one or more acts may be omitted and/or one or more additional acts may be included.

At 1602, an object and/or subject is scanned with an imaging system, which includes direct conversion material based photon counting detectors, producing projection data indicative of the scanned object and/or subject.

At 1604, one or more output photon count rate to input photon count rate maps are obtained in which a map includes at least two input photon count rates for each output photon count rate.

At 1606, one of the at least two input photon count rate is identified as the correct input photon count rate for each detector pixel each integration period using one or more of the embodiments described herein.

At 1608, the projection data is reconstructed based on the identified input photon count rates.

The invention has been described with reference to the preferred embodiments. Modifications and alterations may occur to others upon reading and understanding the preceding detailed description. It is intended that the invention be constructed as including all such modifications and alterations insofar as they come within the scope of the appended claims or the equivalents thereof.

**Claims**

1. An imaging system (600), comprising:

a radiation source (608) that emits polychromatic radiation that traverses an examination region;
a detector array (610) located opposite the radiation source, across the examination region, which includes a paralyzable photon counting detector pixel (611) that detects photons of the radiation that traverse the examination region and illuminate the detector pixel and that generates a signal indicative of each detected photon;
an output photon count rate to input photon count rate map (626) that includes at least one map which maps multiple input photon count rates of the detector pixel to a single output photon count rate of the detector pixel;
an input photon count rate determiner (624) that identifies one input photon count rate of the multiple input photon count rates of the map as a correct input photon count rate for the detector pixel; and
a reconstructor that reconstructs the signal based on the identified input photon count rate.

2. The imaging system of claim 1, the input photon count rate determiner, comprising:

a shaper (1100) that receives the signal and generates pulses for the detected photons, wherein each pulse has a peak amplitude indicative of an energy of the corresponding detected photon;
a comparator (1102) that compares an amplitude of an output of the shaper with a pulse pile-up threshold (1104) and outputs a value indicative of whether the amplitude is below or exceeds the pulse pile-up threshold, wherein the pulse pile-up threshold has a value that corresponds to an energy greater than a highest emission energy of the radiation source;
a counter (1106) that counts a number of times an output of the comparator indicates the amplitude rises above the pulse pile-up threshold within each integration period and outputs a pile-up count value indicative thereof; and
logic (1108) that compares the pile-up count value with a pulse pile-up level threshold (1110) and generates data that indicates whether the pile-up count value is below or above the pulse pile-up level threshold based on the comparison, wherein the data identifies the one input photon count rate of the multiple input photon count rates.

3. The imaging system of claim 1, the input photon count rate determiner, comprising:

a first processing chain (1206), including

a first shaper (1210) that receives a first signal produced by a first detector pixel in response to the first detector pixel detecting a first plurality of detected photons, wherein the first shaper outputs first pulses indicative of the energies of the first plurality of detected photons during an integration period;
a first comparator (1214) that compares an amplitude of the first pulse with a pulse detection threshold (1215) and outputs a first pulse detection signal indicative whether an amplitude of the first pulse exceeds the pulse detection threshold; and
a first counter (1218) that counts a number of times the output of the first comparator indicates the amplitude exceeds the pulse detection threshold and outputs a first output photon count rate;

a second processing chain (1208), including

a second shaper (1212) that receives a second signal produced by a second detector pixel in response to the second detector pixel detecting a second plurality of detected photons, wherein the second shaper outputs second pulses indicative of the energies of the second plurality of detected photons during the integration period, wherein the second detector pixel has a smaller radiation sensitive detection area relative to the first detector pixel,
a second comparator (1216) that compares an amplitude of the second pulse with the pulse detection threshold and outputs a second pulse detection signal indicative whether an amplitude of the second pulse exceeds the pulse detection threshold; and
a second counter (1220) that counts a number of times the output of the second comparator indicates the amplitude exceeds the pulse detection threshold and outputs a second output photon count rate; and
logic (1222) that identifies the input photon count rate based on the first output photon count rate and the second output photon count rate.

4. The imaging system of claim 3, wherein the first and second detector pixels are the same detector pixel or are different detector pixels.

5. The imaging system of claim 3, wherein the logic further determines the correct input photon count rate based on a ratio of a size of the radiation sensitive detection area of first detector pixel to a size of the radiation sensitive detection area of the second detector pixel.

6. The imaging system of claim 1, the input photon count rate determiner, comprising:

a shaper (1300) that receives the signal for a detected photon and generates a pulse indicative of an energy of the detected photon for a plurality of detected photons during the integration period;
a shaper controller (1306) that switches a shaping time of the shaper between at least two different shaping times between two consecutive integration periods;
a comparator (1302) that compares an amplitude of an output of the shaper with a threshold and outputs a signal indicative whether the amplitude exceeds the threshold;
a counter (1106) that counts a number of times the output of the comparator indicates the amplitude exceeds the threshold and outputs a first output photon count rate for a first shaping time of the at least two different shaping times and a second output photon count rate for a second shaping time of the at least two different shaping times; and
logic (1308) that identifies the input photon count rate based on first shaping time, the second shaping time, the first output photon count rate, and the second output photon count rate.

7. The imaging system of claim 1, the input photon count rate determiner, comprising:

a first shaper (1300) that receives the signal for a detected photon and generates a pulse indicative of an energy of the detected photon for a plurality of detected photons during the integration period, wherein the first shaper has a first shaping time;
a second shaper (1300) that receives the signal for a detected photon and generates a pulse indicative of an energy of the detected photon for a plurality of detected photons during the integration period, wherein the second shaper has a second shaping time, and the first and the second shaping times are different;

one or more comparators (1302) that compare an amplitude of an output of the first shaper with a first threshold and an amplitude of an output of the second shaper with a second threshold and respectively outputs first data indicative whether the amplitude of the first signal exceeds the first threshold and second data indicative whether the amplitude of the second signal exceeds the second threshold;

one or more counters (1106) that counts a number of times the output of the one or more comparators indicates the amplitude of the first signal exceeds the first threshold and outputs a first output photon count rate for the first shaping time and counts a number of times the output of the one or more comparators indicates the amplitude of the second signal exceeds the second threshold and outputs a second output photon count rate for the second shaping time; and

logic (1308) that identifies the input photon count rate based on first shaping time, the second shaping time, the first output photon count rate, and the second output photon count rate.

**8.** The imaging system of claim 1, further comprising:

a radiation source controller (1406) that switches a flux of the radiation source between at least two different x-ray fluxes between two consecutive integration periods, wherein the detector array detects photons for a first flux of the at least two different x-ray fluxes and detects photons for a second flux of the at least two different x-ray fluxes;

a shaper (1400) that receives an output of the detector array and generates a pulse indicative of an energy of the detected photon for a photon corresponding to the first flux and a photon corresponding to the second flux;

a comparator (1402) that compares an amplitude of an output of the shaper with a threshold and outputs a signal indicative whether the amplitude exceeds the threshold;

a counter (1404) that counts a number of times the output of the comparator indicates the amplitude exceeds the threshold and outputs a first output photon count rate for the first flux and a second output photon count rate for the second flux; and

logic (1222) that identifies the input photon count rate based on the first output photon count rate, the second output photon count rate, and a ratio of the first flux to the second flux.

**9.** A method, comprising:

receiving an output signal of a paralyzable photon counting detector pixel that is receiving photons at an input photon count rate;

shaping the output signal via a shaper, producing a shaper output signal;

determining an output photon count rate of the detector pixel based on the shaper output signal;

identifying an input photon count rate, from multiple candidate input photon count rates for the output photon count rate, as the input photon count rate corresponding to the detector pixel and the output photon count rate; and

reconstructing the output signal based on the identified input photon count rate.

**10.** The method of claim 9, further comprising:

determining a first output photon count rate corresponding to a first detector pixel for an integration period;

determining a second output photon count rate corresponding to a second detector pixel for the same integration period, wherein the second detector pixel has a radiation sensitive area that is larger than a radiation sensitive area of the first detector pixel; and

determining the input photon count rate based on the first output photon count rate, the second output photon count rate and a ratio of a size of the radiation sensitive area of the first detector pixel to a size of the second larger radiation sensitive area of the second detector pixel.

**11.** The method of claim 9, further comprising:

determining a first output photon count rate for a first shaping time for an integration period;

determining a second output photon count rate for a second shaping time for one of the same or a different integration period, wherein the first and second shaping times are different; and

determining the input photon count rate based on the first output photon count rate, the second output photon count rate, the first shaping time and the second shaping time.

**12.** The method of claim 9, further comprising:

determining a first output photon count rate for a first radiation source flux for an integration period with a first predetermined length;

determining a second output photon count rate for a second radiation source flux for an integration period with a second predetermined length, wherein the first and second fluxes are different; and

determining the input photon count rates based on the first output photon count rate and the second output photon count rate.

**13.** The method of claim 9, further comprising:

> binning detected photons across a plurality of energy bins, wherein each detected photon is binned based on a corresponding energy of the detected photon; and
> determining the input photon count rate based on a distribution of a counted numbers of photons in at least one energy bin.

**14.** The method of claim 13, wherein the distribution is determined based on one or more of an air scan using different and known flux rates or a series of calibration scans using various thicknesses of tissue equivalent materials of known attenuation properties.

**15.** The method of claim 9, further comprising:

> determining the input photon count rate for each data point of a sinogram by assigning data points at a periphery of the sinogram to a higher input photon count rate and assigning data points at a center region of the sinogram to a lower input photon count rate.

FIG. 1

FIG. 2

FIG. 3

FIG. 4

FIG. 5

FIG. 6

624

Input photon count rate determiner

Counter — 706

710

Logic

700

702

708

Shaper → Comparator → Timer

$TH_{TOTL}$

704 — $TH_{PDI}$

IP trigger signal

712

FIG. 7

704

FIG. 8

704

FIG. 9

1000

1002

$TH_{IPCRL}$

$r_{max}$

1004

FIG. 10

14

Input photon count rate determiner 624

1100 1102 1106 1108

Shaper → Comparator → Counter → Logic

1104 — TH$_{PPI}$

IP trigger signal

TH$_{PPL}$ — 1110

FIG. 11

Input photon count rate determiner 624

1202

1210 1214 1218 1206

Shaper → Comparator → Counter

1222

1215 — TH$_{PD}$

IP trigger signal

Logic

1204

Shaper → Comparator → Counter

1208

1212 1216 1220

FIG. 12

EP 3 088 918 A2

Input photon count rate determiner

624

1300 Shaper · 1302 Comparator · 1304 Counter · 1308 Logic

1306 Shaper controller

TH

IP trigger signal

**FIG. 13**

600 Source controller · 1406

Input photon count rate determiner

624

1400 Shaper · 1402 Comparator · 1404 Counter · 1408 Logic

TH

IP trigger signal

**FIG. 14**

EP 3 088 918 A2

FIG. 15

```
┌─────────────────────────────────────┐
│   Scan object and/or subject with an │ ⟋1602
│   imaging system that includes a direct│
│ conversion based photon counting detector│
└─────────────────────────────────────┘
                    │
                    ▼
┌─────────────────────────────────────┐
│   Obtain an output photon count rate │ ⟋1604
│      to input photon count rate map(s)│
└─────────────────────────────────────┘
                    │
                    ▼
┌─────────────────────────────────────┐
│     Identify one of the multiple candidate│ ⟋1606
│ input photon count rates for each detector│
│     pixel each integration period as the│
│       correct input photon count rate│
└─────────────────────────────────────┘
                    │
                    ▼
┌─────────────────────────────────────┐
│     Reconstruct the projection data  │ ⟋1608
│        from the scan based on the    │
│     identified input photon count rate│
└─────────────────────────────────────┘
```

# FIG. 16